Europäisches Patentamt

European Patent Office

Office européen des brevets

(11) Publication number: **0 160 286 B1**

(12) **EUROPEAN PATENT SPECIFICATION**

(45) Date of publication of patent specification: **14.11.90**

(51) Int. Cl.⁵: **A 61 K 9/50**

(21) Application number: **85105142.5**

(22) Date of filing: **26.04.85**

(54) Liposomes.

(30) Priority: **28.04.84 JP 85182/84**

(43) Date of publication of application:
**06.11.85 Bulletin 85/45**

(45) Publication of the grant of the patent:
**14.11.90 Bulletin 90/46**

(84) Designated Contracting States:
**CH DE FR GB LI NL SE**

(56) References cited:
CHEMICAL ABSTRACTS, vol. 95, no. 1, 6th July
1981, page 237, no. 2242r, Columbus, Ohio, US;
H.-W. LEUNG et al.: "The cooperative
interaction between vitamin E and vitamin C in
suppression of peroxidation of membrane
phospholipids"

The file contains technical information
submitted after the application was filed and
not included in this specification

(73) Proprietor: **TERUMO KABUSHIKI KAISHA
trading as TERUMO CORPORATION**
44-1, 2-chome, Hatagaya Shibuya-Ku
Tokyo 151 (JP)

(72) Inventor: **Aoyagi, Juuro**
9-11, 3-chome Hamadayama
Suginami-ku Tokyo (JP)
Inventor: **Yamamoto, Yuichi**
398, Yumisawa-cho
Fujinomiya-shi Shizuoka-ken (JP)
Inventor: **Akaike, Toshihiro**
15-23, 4-chome Shimohoya
Hoya-shi Tokyo (JP)
Inventor: **Nozaki, Yasuhiro**
2-302, Biwakomisoradainidanchi 1-3, Misora-cho
Ootsu-shi Shiga-ken (JP)

(74) Representative: **Henkel, Feiler, Hänzel & Partner**
Möhlstrasse 37
D-8000 München 80 (DE)

Courier Press, Leamington Spa, England.

# EP  0 160 286  B1

(56) References cited:

CHEMICAL ABSTRACTS, vol. 80, no. 3, 21st
January 1974, page 455, no. 15160c, Columbus,
Ohio, US; & JP - A - 73 67 268

CHEMICAL ABSTRACTS, vol. 77, no. 15, 9th
October 1972, page 478, no. 102130z, Columbus,
Ohio, US; & JP - A - 72 25 344

CHEMICAL ABSTRACTS, vol. 71, no. 11, 15th
September 1969, page 457, no. 50463q,
Columbus, Ohio, US; & JP - A - 69 00 218

**Description**

Background of the Invention

Field of the Invention

This invention relates to improved liposomes. More particularly, the invention is concerned with liposomes which are strengthened in their membrane. Liposomes are closed vesicles consisting of membrane comprising bimolecular lipid layers, and are widely used as model membrane of living cellular membrane in the study of physicochemical properties thereof. Furthermore, liposomes are utilized as carriers for administering substances to target organs of living body since they can keep various substances penned up in an interior aqueous compartment or membrane thereof or fuse with cells or are incorporated thereinto. Research heretofore directed to utilization of liposomes has covered a diversity of scientific fields to which liposomes are applicable, for example, biology, medical science and pharmacology, wherein the liposomes are utilized as carriers for transporting enzymes or antitumor agents, in the field of immunology, for examination of interaction thereof with cells, and as drug delivery systems.

Description of the Prior Art

As mentioned hereinbefore, liposomes are of very wide application. As often pointed out, however, the problem involved in liposomes is such that the membranes thereof are fragile. That is, the membrane of liposomes is destroyed by chemical or physical change of a lipid, which is a membrane-forming substance, thereby causing leakage of the substance retained by the liposome. Under these circumstances, as is well known, there have heretofore been proposed various processes for strengthening the membrane of liposomes such as a process in which sphingomeylin is incorporated into the membrane so as to impart hydrogen bonding and strengthen said membrane, and a process in which such additives as tocopherol are incorporated into the membrane so as to prevent oxidation of the unsaturated lipid forming said membrane. These processes, however, are not found sufficiently satisfactory, and there has been a growing demand for an advent of a highly efficient process for strengthening the membrane of liposomes.

Summary of the Invention

Extensive studies carried out by the present inventors with the view of strengthening the membrane of liposomes, resulted in the finding that the liposome membrane is strengthened by adding an ascorbic acid ester to the liposome constituting lipid, wherein the ascorbic acid ester thus added enters in between the bimolecular lipid layers to strengthen hydrogen bonding as well as hydrophobic bonds thereof, and that when the lipid used contains an unsaturated fatty acid, the presence of the ascorbic acid ester thus added prevents oxidation of said lipid and thereby to strengthen the liposome membrane. The present invention has been accomplished on the basis of the above finding.

It is an object of the present invention to provide liposomes having strengthened membrane.

Brief Description of the Drawings

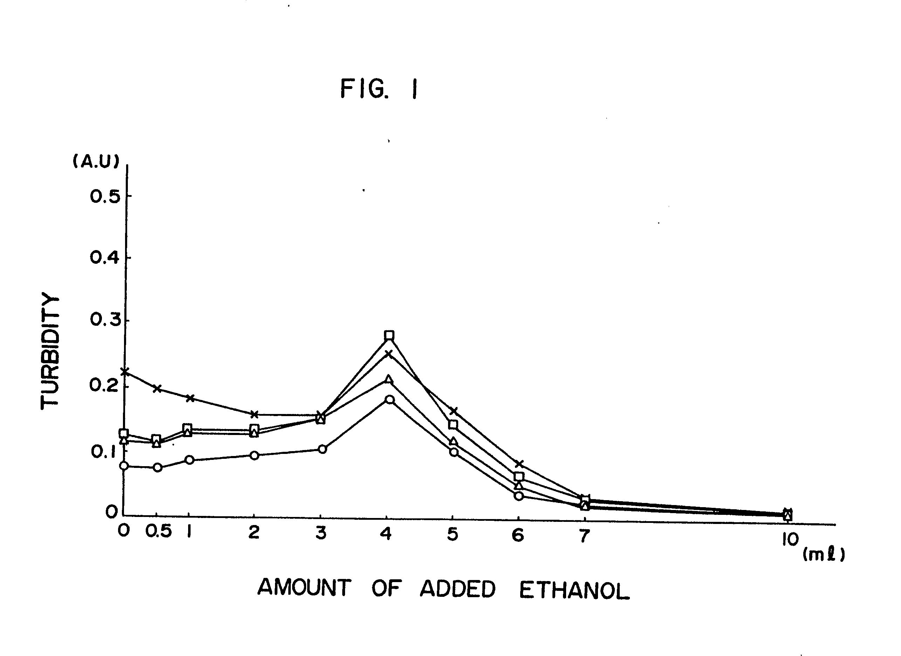

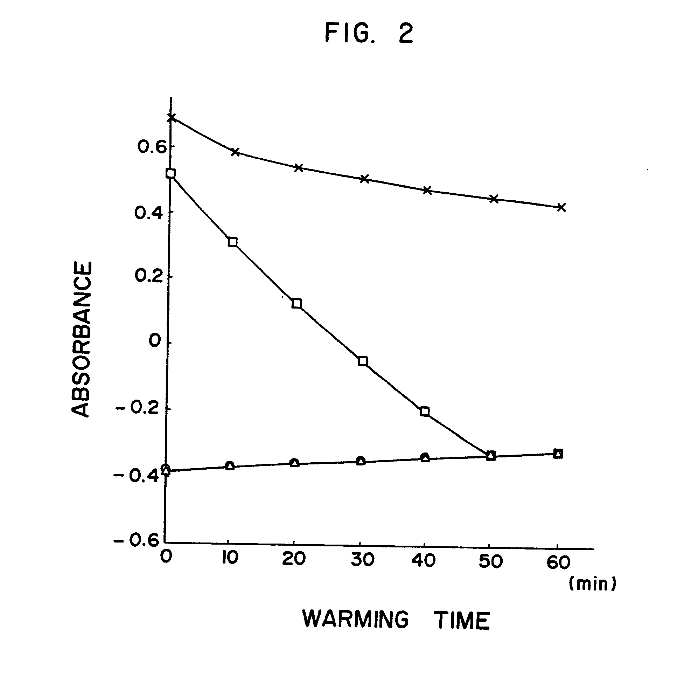

In the accompanying drawings, Fig. 1 is a graph showing ethanol resistance of liposomes according to the present invention, and Fig. 2 is a graph showing anti-oxidation properties of liposomes according to the present invention.

In the graphs of Figs. 1 and 2, respectively, the lines shown below have their respective meanings as indicated.

X—X Composition 1 of the present invention
O—O Composition 2 of the present invention
□—□ Composition 3 of the present invention
Δ—Δ Control

Detailed Description of the Invention

In the liposomes of the present invention, the membane constituting the liposome comprises a lipid containing 0.1—20 mol% (i.e. 1 to 20 mol per 100 mol of said lipid of an ascorbic acid ester.

Usable as lipids constituting liposome membrane in the present invention are either natural or synthetic lipids, without particular limit, so long as they are capable of forming the liposome. In the case where the lipid contains in the molecule an unsaturated fatty acid, oxidation of said lipid is prevented. Preferably usable as lipids in the present invention are phospholipids, and examples of the phospholipids which may be used either alone or in combination include lecithin (phosphatidyl choline), phosphatidyl ethanolamine, phosphatidic acid, phosphatidyl serine, phosphatidyl inositol, phosphatidyl glycerol, sphingomyelin, cardiolipin, and those hydrogenated according to the usual method. In particular, phosphatidyl choline or sphingomyelin is preferred among these phosholipids.

Ascorbic acid esters used in the present invention should be fat-soluble, and preferably are mono- or diesters of an ascorbic acid with fatty acids, having 12 to 22 carbon atoms, particularly 14 to 18 carbon atoms. In particular, palmitic acid and stearic acid are preferred.

The amount of an ascorbic acid ester used is suitably 0.1 to 20 mol%, i.e. the amount of the ester used is 0.1—20 mol per 100 mol of the lipid used. The ascorbic acid esters can be qualitatively analyzed by thin layer chromatography and determined by liquid chromatography. No strengthening of membrane can be attained if a concentration of the ester used is lower than 0.1 mol%. The use of the ester in a concentration higher than 20 mol% is generally not desirable since physiological activity of ascorbic acid is exhibited simultaneously with strengthening of membrane.

For enhancing the membrane in strength, it is possible to add sterols such as cholesterol to the liposome of the present invention. In order to regulate slow releasing property of medicine kept penned in the present liposome in living body, moreover, it is possible to add charge donating substances, for example, phosphatidic acid, dicetyl phosphate, stearylamine, etc. to the present liposome. Possible destruction of the liposome membrane may be controlled by the presence of these substances mentioned above.

The liposome, per se, of the present invention or those having penned therein various substances are utilized in a wide variety of fields.

For instance, the present liposomes may be used as cell splitting materials, utilizing their affinity for living cellular membrane. Moreover, the present liposomes may be utilized as carriers for medicines which are unstable in vitro or in vivo, or which are desired to be gradually released in living body or to be distributed quickly to specific target organs. Examples of the medicines referred to above include insulin, heparin, urokinase, ubidecarenone, methotrexate, neomycin, bleomycin, tetracycline, cytochrome C, asparaginase, cytosine and arabinoside. In addition thereto, no particular limitation is placed on substances other than medicines which are intended to be carried by the present liposomes so long as they may be effectively administered to the living body, such as markers, plasmids, DNA or RNA.

The liposomes of the present invention may be prepared by methods known, per se.

For instance, a lipid constituting liposome and an ascorbic acid ester, and, if desired, a sterol, a charge donating substance and a fat-soluble medicine, are dissolved in a suitable organic solvent such as chloroform or ethanol. The resulting solution is evaporated to remove the solvent therefrom and thereby to prepare a thin membrane of the lipid. To the thin membrane is added an aqueous solution of a substance which is desired to be incorporated into said thin membrane, followed by vigorous stirring, whereupon there is formed a multilayer liposome. Supersonic treatment of the multilayer liposome gives a liposome of about 25—50 nm (250Å—500Å) in diameter.

Where the liposome as prepared is intended to be used as a medicine-retaining liposome, said liposome is washed, if necessary, with for example, physiological saline water, and then formulated into a pellet, suspended preparation, tablet, capsuled preparation, granule preparation, or dust preparation for oral administration, or formulated into an injectable preparation for parenteral administration.

The liposomes of the present invention have a membrane composed of a lipid containing 0.1—20 mol% of an ascorbic acid ester. In the liposomes, the membrane is enhanced in strength because the ascorbic acid ester used enters between the bimolecular lipid layers of the membrane, whereby hydrogen bonding is strengthened.

Appearance of double concentric circles under a transmission type electron microscope proves that the liposomes comprise bimolecular lipid layers.

Furthermore, in the case where the present liposomes have lipids containing unsaturated fatty acids, oxidative degradation of said lipids is prevented by the presence of the ascorbic acid ester used, with the result that the present liposomes have excellent storage stability.

The present invention is illustrated more fully below with reference to example and experimental example.

## Example

Liposomes having their respective composition as indicated in Table 1 were prepared according to the back phase method (Proc. Natl. Acad. Sci. USA. 75(9). 4194 (1978); Japanese Patent Laid-Open-to-Public Publn. No. 118415/1980).

A solution of predetermined amounts of egg phosphatidyl choline, cholesterol, ascorbic acid esters, sphingomyelin, and α-tocopherol in 20 ml of chloroform was placed in a 50 ml Erlenmyer's flask. Using a rotary evaporator, the solvent was distilled off to form a thin membrane on the inner wall of the flask.

Subsequently, 18 ml of diethyl ether was added to the thin membrane in the flask to dissolve the lipid and thereto was added 3 ml of a Tyrode's buffer from which $Ca^{2+}$, $Mg^{2+}$, and glucose has been excluded. Then, after the gaseous phase in the interior of the flask was replaced with nitrogen gas, the flask was irradiated with ultrasonic waves for about 5 minutes, while maintaining the flask at about 20°C, until a uniform suspension was formed. The greater part of the diethyl ether was distilled off at 20—25°C under reduced pressure (reduced gauge pressure about 53,2 kPa = 400 mmHg) using a rotary evaporator. After mixing the gel-like lipid obtained in the matter now described for 10—15 seconds with Bortex®, the diethyl ether was completely removed at 20—25°C under reduced pressure (reduced gauge pressure about 97,1 kPa = 730 mmHg) therefrom.

The lipid was suspended again in a Tyrode's buffer in the Erlenmyer's flask, from which $Ca^{2+}$, $Mg^{2+}$, and glucose had been excluded, and after replacing the gaseous phase in the interior of the flask with nitrogen gas, the flask was irradiated at about 20°C for 30 minutes with ultrasonic waves to collect vesicles of the liposome of a single layer or oligolamella structure.

TABLE 1

Liposome composition (mol ratio)

|  | EPC | Chol | AP | ADP | AS | SPIN | VE |
|---|---|---|---|---|---|---|---|
| Present invention Composition 1 | 8.1 | 10.0 | 2.0 | — | — | | |
| Present invention Composition 2 | 8.0 | 10.0 | — | 2.0 | — | | |
| Present invention Composition 3 | 8.1 | 10.0 | — | — | 2.0 | | |
| Control | 8.1 | 10.0 | — | — | — | 2.0 | 0.3 |

Total lipid concentration 18 μmol/ml

| EPC | : | Egg phosphatidyl choline |
|---|---|---|
| Chol | : | Cholesterol |
| AP | : | Ascorbyl 6-palmitate |
| ADP | : | Ascorbyl 2,6-dipalmitate |
| AS | : | Ascorbyl 6-stearate |
| SPN | : | Sphingomyelin |
| VE | : | α-tocopherol (vitamin E) |

Experimental Example

The liposomes obtained in the above-mentioned Example were tested for ethanol resistance and antioxidation properties.

(1) Ethanol resistance test

The test was carried out in accordance with the method described in S. L. Regen, et. J. Am. Chem. Soc., 102, 6638—6640 (1980).

To 5 ml of a 10-fold diluted solution of liposomes (diluted with a Tyrode's buffer from which $Ca^{2+}$, $Mg^{2+}$, and glucose had been excluded) was successively added ethanol in amounts of 0.5, 1.0, 2.0, 3.0, 4.0, 5.0, 6.0, 7.0, and 10.0 ml respectively, and at each time of the addition the diluted solution was measured for turbidity at wavelength of 400 nm. In the graph, showing turbidity on the vertical axis and the amount of ethanol added on the horizontal axis, each measured value was plotted to obtain the gradient of the linear regression line between 0—2 ml of the amount of ethanol added. The results obtained are shown in Table 2 and Fig. 1. The smaller the absolute value of gradient, the greater the ethanol resistance, showing a stronger membrane structure.

(2) Antioxidation properties test

The test was carried out according to the method described in "Chemistry of Lipids", compiled by Japan Biochemistry Society, p. 538—539, 1974 (Tokyo Kagaku Dohin).

To 0.5 ml of the liposome was added 2.5 ml of 0.2M acetate buffer followed by 5 ml of a $10^{-4}$ M 1,1-diphenyl-2-picrylhydrazyl solution (methanol/water = 4:1 mixture), and the resulting mixture was thoroughly stirred. The mixture was incubated at 40—50°C, and absorbance at a wavelength of 517 nm was measured every ten minutes over 60 minutes.

In the graph, showing absorbance on the vertical axis and the warming time on the horizontal axis, each measured value was plotted to obtain gradient of the linear regression line during the warming time of from 10 to 60 minutes. The results are shown in Table 3 and Fig. 2. The smaller the absolute value of the gradient, the higher the anti-oxidation properties.

5

TABLE 2

| Sample | Gradient |
|---|---|
| Present invention Composition 1 | 0.011 |
| Present invention Composition 2 | 0.007 |
| Present invention Composition 3 | 0.008 |
| Control | −0.031 |

TABLE 3

| Sample | Gradient |
|---|---|
| Present invention Composition 1 | $9.34 \times 10^{-4}$ |
| Present invention Composition 2 | $-1.59 \times 10^{-2}$ |
| Present invention Composition 3 | $9.71 \times 10^{-4}$ |
| Control | $-2.94 \times 10^{-3}$ |

As is clear from Tables 2 and 3, and from Figs. 1 and 2, the ascorbic acid esters show a greater effect of strengthening the membrane of liposomes than sphingomyelin in the ethanol resistance test, and have the effect of improving antioxidation properties of liposomes equal to that of α-tocopherol.

**Claims**

1. A liposome whose membrane is composed of a lipid containing 0.1—20 mol of an ascorbic acid ester per 100 mol of said lipid.

2. The liposome according to Claim 1 wherein the lipid has an unsaturated bond.

3. The liposome according to Claim 2 wherein the lipid having an unsaturated bond is phospholipid.

4. The liposome according to Claim 3 wherein the phospholipid is phosphatidyl choline or sphingomyelin.

5. The liposome according to Claim 1 wherein the ascorbic acid ester is a mono- or diester of ascorbic acid with a higher fatty acid having 12 to 22 carbon atoms.

6. The liposome according to Claim 5 wherin the said ascorbic acid mono- or diester is ascorbic acid mono- or dipalmitate or ascorbic acid mono- or distearate.

7. The liposome according to Claim 1 wherein the liposome encapsulates a chemical compound.

**Patentansprüche**

1. Liposom, dessen Membran aus einem Lipid mit 0,1—20 Mol eines Ascorbinsäureesters pro 100 Mol Lipid besteht.

2. Liposom nach Anspruch 1, dadurch gekennzeichnet, daß das Lipid eine ungesättigte Bindung enthält.

3. Liposom nach Anspruch 2, dadurch gekennzeichnet, daß das Lipid mit einer ungesättigten Bindung aus eiem Phospholipid besteht.

4. Liposom nach Anspruch 3, dadurch gekennzeichnet, daß es sich bei dem Phospholipid um Phosphatidylcholin oder Sphingomyelin handelt.

5. Liposom nach Anspruch 1, dadurch gekennzeichnet, daß es sich bei dem Ascorbinsäureester um einen Mono- oder Diester von Ascorbinsäure mit einer höheren Fettsäure mit 12—22 Kohlenstoffatomen handelt.

6. Liposom nach Anspruch 5, dadurch gekennzeichnet, daß es sich bei dem Ascorbinsäuremono- oder -diester um Acorbinsäuremono- oder -dipalmitat oder Ascorbinsäuremono- oder -distearat handelt.

7. Liposom nach Anspruch 1, dadurch gekennzeichnet, daß es eine chemische Verbindung einkapselt.

**EP 0 160 286 B1**

**Revendications**

1. Liposome dont la membrane est composée d'un lipide contenant 0,1 à 20 moles d'ester d'acide ascorbique pour 100 moles dudit lipide.

2. Liposome selon la revendication 1, dans lequel le lipide a une liaison insaturée.

3. Liposome selon la revendication 2, dans lequel le lipide ayant une liaison insaturée est un phospholipide.

4. Liposome selon la revendication 3, dans lequel le phospholipide est la phosphatidylcholine ou la sphingomyéline.

5. Liposome selon la revendication 1, dans lequel l'ester d'acide ascorbique est un mono ou diester d'acide ascorbique et d'une acide gras supérieur ayant 12 à 22 atomes de carbone.

6. Liposome selon la revendication 5, dans lequel ledit mono ou diester d'acide ascorbique est le mono ou dipalmitate d'acide ascorbique ou le mono ou distéarate d'acide ascorbique.

7. Liposome selon la revendication 1 qui encapsule un composé chimique.

7

## FIG. 1

EP 0 160 286 B1

FIG. 2